# EUROPEAN PATENT APPLICATION

(11) **EP 4 427 714 A1**
(43) Date of publication of application: **11.09.2024**
(21) Application number: 22889968.8
(22) Date of filing: 02.11.2022
(51) Int. Cl.: A61F 7/12, A61M 5/142

(54) **IN-VIVO TEMPERATURE CONTROL SYSTEM**

(30) Priority: 04.11.2021 JP 2021179995
(71) Applicant: Toray Industries, Inc., Tokyo 103-8666 (JP)
(72) Inventor: SAKAKIBARA, Hajime, Otsu-shi, Shiga 520-2141 (JP); NAKAJIMA, Hiroki, Tokyo 103-8666 (JP); MATSUKUMA, Akinori, Otsu-shi, Shiga 520-2141 (JP); MIKAMI, Jun, Otsu-shi, Shiga 520-2141 (JP)
(74) Representative: Kador & Partner Part mbB
(86) International application number: PCT/JP2022/040957
(87) International publication number: WO 2023/080148

(57) **Abstract**

The present invention aims to provide an *in-vivo* temperature control system capable of adjusting a temperature in a biological lumen by continuously or intermittently discharging a liquid into the biological lumen such as the esophagus and collecting and eliminating the released liquid from a living body to the outside. The present invention provides the *in-vivo* temperature control system including: a first tube provided with a first channel; a second tube provided with a second channel; a liquid storage section for storing the liquid; a liquid injection pump for supplying the liquid from the liquid storage section to the first channel and discharging the liquid into a living body via the first channel; a liquid suction pump for collecting the liquid in the living body via the second channel; and a third tube provided with a third channel, one end of which is connected to the liquid suction pump, and the other end of which is opened to the outside. The liquid suction pump switches operation between discharge operation for discharging the liquid in the living body into the third channel and release operation for releasing a pressure in the second channel to the outside via the third channel.

## Description

### TECHNICAL FIELD

The present invention relates to an *in-vivo* temperature control system.

### BACKGROUND ART

Atrial fibrillation is a type of arrhythmia and is known as a cause of discomfort and fatigue triggered by poor blood circulation resulted from repeated irregular contraction of the atrium. To handle the above, a method for treating the atrial fibrillation has been widely carried out by a catheter ablation procedure (pulmonary vein isolation) for ablating pulmonary veins, which are major sources of the atrial fibrillation, and nearby myocardial tissue in a posterior wall or the like of the left atrium.

Meanwhile, since the ablation site (left atrium) and the esophagus are positioned close to each other, it has been pointed out that the treatment by the catheter ablation procedure has a risk of damaging the esophagus, leading to severe esophageal complications such as left atrial-esophageal fistula and esophagus vagus nerve paralysis. Thus, appropriate control for the internal esophageal temperature has been demanded.

As means for controlling the internal esophageal temperature, a temperature measurement device has been reported that employs an approach through a patient nose or a patient mouth (nasal or oral approach) to insert a catheter equipped with a temperature sensor into the esophagus and measure the internal esophageal temperature. Then, when it is determined that the internal temperature has reached a threshold, the temperature measurement device outputs an alert to the outside (Patent Document 1).

In addition, an esophageal catheter system has been reported in which first and second expansion members are brought into contact with the esophageal wall and a liquid is injected between the expansion members to control the internal esophageal temperature (Patent Document 2).

Furthermore, as means for collecting the liquid from inside of a living body, an ultrasound treatment system has been reported that has a suction pipeline for suctioning and removing unwanted substances, including blood and tissue fragments, produced by ultrasonic treatment such as crushing, excising, or emulsifying biological tissue, a stone, or the like (Patent Document 3).

### PRIOR ART DOCUMENTS

### PATENT DOCUMENTS

[Patent Document 1] JP 6618254 B
[Patent Document 2] JP 2019-80783 A
[Patent Document 3] JP 3065721 B

### SUMMARY OF THE INVENTION

### PROBLEMS TO BE SOLVED BY THE INVENTION

In order to appropriately control the internal esophageal temperature during the treatment by the catheter ablation procedure, it is required to continuously monitor the internal esophageal temperature and take a proactive measure before intraesophageal tissue is damaged.

The temperature measurement device described in Patent Document 1 monitors the internal esophageal temperature during the ablation treatment, and outputs the alert to the outside when it is determined that the internal temperature has reached the threshold. In this way, the temperature measurement device can take the proactive measure, such as pausing the ablation, before the esophagus is damaged by heating or cooling. However, the esophagus temperature may be rapidly changed during the ablation treatment. Thus, when there is a delay in noticing the alert, a necessary measure may not be taken in a timely manner. For this reason, it is desired to control the internal esophagus temperature by liquid injection or the like.

The esophageal temperature control system described in Patent Document 2 can appropriately control the internal esophageal temperature by forming a space between the two expansion members, injecting the liquid into the space while plugging, and collecting the liquid therefrom. However, in the case where the injected liquid is continuously suctioned from the inside of the esophagus, but there is no more liquid left to be suctioned, the esophageal wall is drawn toward an opening and closes the opening, resulting in a negative pressure generated in a liquid elimination channel. In the above state, the liquid cannot be collected from the esophagus until drawing of the esophageal wall to the opening is eliminated. Thus, in order to continuously suction the liquid from the inside of the esophagus, it is desired that the pressure in the liquid elimination channel can be released each time.

The ultrasound treatment system described in Patent Document 3 is equipped with an ultrasonic treatment probe having a suction channel, a suction pipeline connected to the suction channel of this ultrasonic treatment probe, a suction pump connected to the suction channel of the probe through the suction pipeline, a suction container inserted in the middle of the suction pipeline to remove a suctioned substance, a leak pipeline as a separate component from the suction pipeline and communicating between an upper space in the suction container and outside air, and a leak valve for opening the leak pipeline during leakage. By opening the leak valve, it is possible to prevent the procedure site from remaining suctioned to a distal end of the ultrasonic treatment probe by the negative pressure when the liquid is suctioned from the inside of the living body. However, the leak pipeline and the leak valve for releasing the pressure have to be provided separately from the suction pipeline, which increases the number of components.

In view of the above, the present invention aims to provide an in-vivo temperature control system having means for adjusting a temperature in a biological lumen while preventing suction failure caused by a negative pressure with a simple configuration in which a liquid is discharged continuously or intermittently into the biological lumen such as an esophagus and operation for collecting the discharged liquid from a living body and eliminating the liquid to the outside is linked with operation for releasing the pressure in a liquid elimination channel.

### MEANS FOR SOLVING THE PROBLEMS

In order to solve the above problem, the present inventors intensively studied to discover the following inventions (1) to (5):
(1) An *in-vivo* temperature control system, comprising:
   a first tube provided with a first channel;
   a second tube provided with a second channel;
   a liquid storage section for storing a liquid;
   a liquid injection pump for supplying the liquid from the liquid storage section to the first channel and discharging the liquid into a living body via the first channel;
   a liquid suction pump for collecting the liquid in the living body via the second channel; and
   a third tube provided with a third channel, one end of which is connected to the liquid suction pump, and the other end of which is opened to the outside,
   wherein the liquid suction pump switches operation between discharge operation for discharging the liquid in the living body into the third channel and release operation for releasing the pressure in the second channel to the outside via the third channel.
(2) The *in-vivo* temperature control system of (1), comprising a shaft inserted into the first tube and protruding from the distal end of the first tube.
(3) The *in-vivo* temperature control system of (1) or (2), comprising:
   a temperature sensor arranged in the first tube, the second tube, or the shaft and capable of measuring a temperature in the living body; and
   a control section for controlling the liquid injection pump and the liquid suction pump,
   wherein the control section controls the liquid injection pump to discharge the liquid from the first tube into the living body on the basis of the temperature measured by the temperature sensor, and controls the liquid suction pump to switch the operation between the discharge operation and the release operation on the basis of a drive record of the liquid injection pump.
(4) The *in-vivo* temperature control system of any one of (1) to (3),
   wherein the liquid suction pump has a roller pump,
   in the discharge operation, the second channel is pressed by rotational drive of a roller to deliver the liquid in the second tube, and
   in the release operation, the pressure in the second channel is released to the outside by providing an unpressed state of the second channel during one rotation of the roller.
(5) The *in-vivo* temperature control system of any one of (1) to (3),
   wherein the liquid suction pump has a finger pump including a plurality of fingers,
   in the discharge operation, the plurality of fingers sequentially press a fixed range of the second channel to deliver the liquid in the second tube, and
   in the release operation, the pressure in the second channel is released to the outside by providing an unpressed state of the second channel during delivery of the liquid by the fingers.

   In order to solve the above problem, the present inventors intensively studied to discover the following inventions (6) to (9):
(6) An *in-vivo* temperature control system, comprising:
   a first tube provided with a first channel;
   a second tube provided with a second channel;
   a liquid storage section for storing a liquid;
   a liquid injection pump for supplying the liquid from the liquid storage section to the first channel and discharging the liquid into a living body via the first channel;
   a liquid suction pump for collecting the liquid in the living body via the second channel; and
   a third tube provided with a third channel, one end of which is connected to the liquid suction pump, and the other end of which is opened to the outside,
   wherein the liquid suction pump switches operation between discharge operation for discharging the liquid in the living body into the third channel and release operation for releasing a pressure in the second channel to the outside via the third channel.
(7) The *in-vivo* temperature control system of (6), comprising:
   a temperature sensor arranged in the first tube or the second tube and capable of measuring a temperature in the living body; and
   a control section for controlling the liquid injection pump and the liquid suction pump,
   wherein the control section controls the liquid injection pump to discharge the liquid from the first tube into the living body on the basis of the temperature measured by the temperature sensor, and controls the liquid suction pump to switch the operation between the discharge operation and the release operation on the basis of a drive record of the liquid injection pump.
(8) The *in-vivo* temperature control system of (6) to (7),
   wherein the liquid suction pump has a roller pump,
   in the discharge operation, the second channel is pressed by rotational drive of a roller to deliver the liquid in the second tube, and
   in the release operation, the pressure in the second channel is released to the outside by providing an unpressed state of the second channel during one rotation of the roller.
(9) The *in-vivo* temperature control system of (6) to (7),
   wherein the liquid suction pump has a finger pump including a plurality of fingers,
   in the discharge operation, the plurality of fingers sequentially presses a fixed range of the second channel to deliver the liquid in the second tube, and
   in the release operation, the pressure in the second channel is released to the outside by providing an unpressed state of the second channel during delivery of the liquid by the fingers.

### EFFECT OF THE INVENTION

According to the present invention, since the pressure in the liquid elimination channel is released in conjunction with the drive of the liquid suction pump, it is possible to prevent tissue in a biological lumen from remaining suctioned to the vicinity of the opening due to suction. Thus, the discharged liquid can be suctioned continuously or intermittently. As it has been described so far, the temperature can be adjusted to a predetermined temperature while the liquid is circulated in the biological lumen. In addition, the number of components can be reduced by eliminating the need for a pressure gauge, a pressure relief valve, and the like in the channels.

### BRIEF DESCRIPTION OF THE DRAWINGS

Fig. 1 is a schematic view illustrating an internal structure of an *in-vivo* temperature control system according to a first embodiment of the present invention.
Fig. 2 includes schematic views illustrating more specific operation of a liquid suction pump in the *in-vivo* temperature control system illustrated in Fig. 1.
Fig. 3 is a schematic view illustrating an internal structure of an *in-vivo* temperature control system according to a second embodiment of the present invention.
Fig. 4 is a graph indicating results of recording pressure fluctuations in second channels by using the in-vivo temperature control systems in an example and a comparative example.

### MODE FOR CARRYING OUT THE INVENTION

Specific embodiments of the present invention are described below with reference to the drawings. However, the present invention is not limited to these modes. The present invention may be modified as appropriate without departing from the scope in which the effect of the present invention can be produced. It should be noted that the same symbols are used for the same elements.

### <First Embodiment>

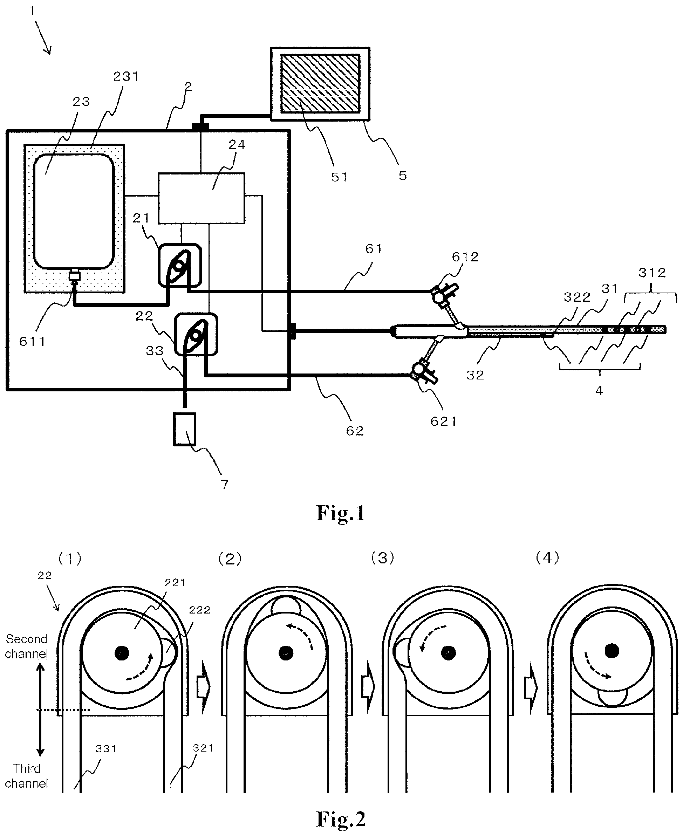

Fig. 1 is a schematic view of an *in-vivo* temperature control system 1 according to a first embodiment. For example, when an ablation procedure is carried out using a balloon ablation catheter in which inside of a balloon is heated using a radiofrequency current, the *in-vivo* temperature control system 1 may be used to monitor an internal esophageal temperature in the vicinity of the heart to be treated by the ablation, and also to reduce the internal esophageal temperature with a liquid. A biological organ to which the *in-vivo* temperature control system 1 is applicable is not particularly limited. The system may be applied to the pharynx, larynx, lung, esophagus, stomach, or the like. It is especially preferably used to cool the inside of the esophagus.

Here, the *in-vivo* temperature control system 1 includes: an *in-vivo* temperature control device 2 having a liquid injection pump 21 for injecting a temperature-controlled liquid into a living body and a liquid suction pump 22 for suctioning the liquid from the inside of the living body; a first tube 3 1 provided with a first channel 311 inserted into the living body to inject the liquid into the living body and an opening 312 leading to the first channel 311; a second tube 32 provided with a second channel 321 inserted into the living body to suction the liquid from the inside of the living body and an opening 322 leading to the second channel 321; a temperature sensor 4 capable of measuring a temperature in the living body; a monitor 5 capable of displaying a signal from the temperature sensor 4; a tube 61 connected the liquid injection pump21 and connecting a liquid storage section 23 to the first tube 31; a tube 62 connecting the liquid suction pump 22 to the second tube 32; and a third tube 33 provided with a third channel 331, one end of which is connected to the second channel 321, and the other end of which is opened to the outside. Here, the liquid eliminated from the third tube 33, which is opened to the outside, can be collected in a waste liquid section 7 as a receptacle via the open outside.

The *in-vivo* temperature control device 2 includes the liquid injection pump 21, the liquid suction pump 22, the liquid storage section 23 for storing the liquid, and a control section 24 for controlling drive of the liquid injection pump 21 and the liquid suction pump 22 on the basis of the signal detected from the temperature sensor 4 or a preset condition.

In the first embodiment, the liquid injection pump 21 can inject the liquid into the living body via the first channel 311, and the liquid suction pump 22 can switch operation between discharge operation for discharging the liquid in the living body to the outside via the second channel 321 and the third channel 331 connected to the second channel and release operation for releasing a pressure in the second channel 321 to the outside via the third channel 331. Preferably, the discharge operation and the release operation described above are alternately switched. In the case where it is structured to perform an operation other than a time for the discharge operation for discharging the liquid in the living body to the outside and the release operation for releasing the pressure to the outside, the other operation may be included during the switching. For example, in the case where the liquid injection pump 21 has a mechanism for performing simultaneous operations of the discharge operation and the release operation, the operation may be switched to the operation for simultaneously performing the discharge operation and the release operation.

A pump drive method used for the liquid injection pump 21 and the liquid suction pump 22 is not limited. However, it is preferred to use a tube pump capable of maintaining cleanliness of the liquid to be delivered/suctioned into/from the living body and easily controlling the flow rate of the liquid, and a tube pump of a roller type or a finger type is further preferred.

When the liquid is suctioned by the liquid suction pump 22 from inside of an easily-deformable biological lumen such as the esophagus, an inner wall of the biological lumen may be drawn toward the opening 322, resulting in a negative pressure generated in the second channel 321 for liquid elimination. In order to prevent the inner wall of the biological lumen from being drawn to the opening 322 while under the negative pressure, the liquid suction pump 22 can release the pressure in the second channel to the outside via the third channel 331. For example, as in the schematic views of Fig. 2 illustrating the more specific operation of the liquid suction pump, the liquid suction pump 22 includes a rotor 221 that is rotationally driven and a roller 222 rotatably supported by the rotor 221. A channel in the pump as an end portion of the second channel 321 is provided in the liquid suction pump 22. In addition, the channel in the pump as the end portion of the second channel 321 is integrally connected to the third channel 331 opened to the outside. As in (1) to (3) of Fig. 2, due to rotational drive operation, the single roller 222 draws the liquid in the second channel 321 using the pressure while the roller 222 presses a portion of the second channel 321 as the channel in the pump. In this way, the roller 222 can perform the discharge operation for discharging the liquid in the living body to the third channel 331. Then, as in (4) of Fig. 2, when the single roller 222 is switched into a state of not pressing the portion of the second channel 321 as the channel in the pump, the inside of the second channel 321 is integrated with the third channel 331 opened to the outside, thereby releasing the pressure in the second channel 321. Thus, the release operation can be performed for releasing the pressure in the second channel 321 to the outside via the third channel 331, and the discharge operation and the release operation can be alternately performed by rotating the single roller 222, just as described. Here, in a case of the roller type, the control section 24, which controls the drive of the liquid suction pump 22, controls a rotational frequency of the rotor 221 and thus can determine times for the discharge operation and the release operation performed alternately.

The single roller 222 is provided in the first embodiment. However, a plurality of the rollers 222 may be provided as long as, in the state of not pressing the second channel 321, the release operation for releasing the pressure in the second channel 321 to the outside via the third channel 331 is attained.

When the roller 222 presses the second channel 321, the second channel 321 does not have to be completely closed, and the liquid can be discharged by arranging the waste liquid section 7 to be lower than the biological lumen, from which the liquid is suctioned, and pushing out the liquid into the waste section 7 by own weight of the liquid. In order to discharge the liquid efficiently, it is preferred that the roller 222 completely press the portion of the second channel 321 as the channel in the pump.

As an embodiment other than the first embodiment, the liquid suction pump 22 may be a finger pump with fingers. Also in the above configuration, the channel in the pump as the end portion of the second channel 321 is provided in the liquid suction pump 22, and the discharge operation for discharging the liquid in the second channel 321 to the third channel 33 1 can be performed by sequentially pressing a fixed range of the portion of the second channel 321 as the channel in the pump with the fingers. Then, by providing an unpressed state of the channel in the pump in of the second channel 321, the inside of the second channel 321 is integrated with the third channel 331 opened to the outside, thereby releasing the pressure in the second channel 321. Thus, the release operation can be performed alternately for releasing the pressure in the second channel 321 to the outside via the third channel 331. In a case of the finger type, the control section 24, which controls the drive of the liquid suction pump 22, controls a time for pressing the portion of the second channel 321 as the channel in the pump with the fingers and thus can determine the times for the discharge operation and the release operation performed alternately.

When the fingers press the second channel 321, the second channel 321 does not have to be completely closed, and the liquid can be discharged by arranging the waste liquid section 7 to be lower than the biological lumen, from which the liquid is suctioned, and pushing out the liquid into the waste section 7 by the own weight of the liquid. In order to discharge the liquid efficiently, it is preferred that the fingers completely press the portion of the second channel 321 as the channel in the pump.

The liquid storage section 23 of the *in-vivo* temperature control system 1 in the first embodiment is a generally, commercially available infusion bag for physiological saline, glucose solution, or the like. The liquid storage section 23 has a structure by which it can be housed in the *in-vivo* temperature control device 2. In the *in-vivo* temperature control device 2, a Peltier device 231 and an in-device temperature sensor, which is not illustrated, for measuring the temperature in the liquid storage section 23 are placed to be in contact with the liquid storage section 23. By controlling the temperature of the Peltier device 231 on the basis of a signal detected from the in-device temperature sensor, the temperature of the liquid stored in the liquid storage section 23 can be controlled. In the case where a catheter ablation procedure using a hot balloon is carried out, the temperature of the liquid in the liquid storage section 23 is preferably controlled at 0°C to 15°C and more preferably controlled at 0°C to 10°C.

The liquid storage section 23 may be in any shape as long as capable of storing the liquid such as the physiological saline. The liquid storage section 23 may be housed in the *in-vivo* temperature control device 2 or may be placed outside the *in-vivo* temperature control device 2. In the case where the liquid storage section 23 is housed in the *in-vivo* temperature control device 2, as described above, the temperature of the liquid in the liquid storage section 23 can be preferably controlled. In an embodiment other than the first embodiment, the infusion bag may be cooled using a coolant such as ice instead of the Peltier device 231, or the infusion bag that has been frozen in advance may be thawed and used. The liquid to be used may be purified water or tap water instead of the physiological saline or the glucose solution.

In a case of use in cryoablation that requires the inside of the living body to be warmed to a higher level than usual, the *in-vivo* temperature control system of the present invention may be used therefor. In such a case, the liquid stored in the liquid storage section 23 may be using a heating resistor.

The control section 24 provided in the *in-vivo* temperature control device 2 detects the signal (such as a thermoelectromotive force) from the temperature sensor 4, and then converts the signal to temperature information (an *in-vivo* temperature).

The control section 24 controls the liquid injection pump 21, and drives the liquid injection pump 21 on the basis of the signal detected from the temperature sensor 4 or an injection command issued by operating the monitor 5 such that the liquid in the liquid storage section 23 is discharged to the outside via the first tube 31.

The control section 24 also controls the liquid suction pump 22, and drives the liquid suction pump 22 on the basis of the signal detected from the temperature sensor 4 or a suction command issued by operating the monitor 5, so as to eliminate the liquid in the living body to the outside via the second tube 32.

The control section 24 preferably includes a circuit for controlling the internal temperature of the liquid storage section 23 on the basis of the signal detected from the in-device temperature sensor for measuring the temperature in the liquid storage section 23.

The first tube 31 and the second tube 32 are tubular members, each of which can be inserted into the living body through the nasal or oral approach, and includes the opening 312 for injecting the liquid into the living body and the opening 322 for suctioning the liquid from the inside of the living body, respectively.

In regard to the opening 312, the inside of the first tube 31, that is, the first channel 311 in the first tube 31 and the outside of the first tube 31 communicate with each other via the opening 312. A plurality of the openings 312 is provided on a side surface of the first tube 31, but may be provided at a distal end of the first tube 31. When the catheter ablation procedure using the hot balloon is carried out, the openings 312 are preferably arranged in the vicinity of the temperature sensor 4. The above configuration allows the rapid injection of the cooled liquid into a hot esophageal site when the temperature in the esophagus is increased due to the ablation procedure.

In regard to the opening 322, the inside of the second tube 32, that is, the second channel 321 in the second tube 32 and the outside of the second tube 32 communicate with each other via the opening 322. Although the opening 322 is provided at the distal end of the second tube 32, one or a plurality of the openings 322 may be provided on a side surface of the second tube 32.

Any flexible material that can be nasally or orally inserted into the living body may be used for the first tube 31 and the second tube 32. For example, a thermoplastic resin such as polyvinyl chloride, polyurethane, or silicone can be used. In addition, in order to check a placement site in the living body, the material preferably contains a radiographic contrast material.

For example, in the case where the first tube 31 and the second tube 32 are nasally inserted into the living body from a nose, the length of the tube section 31 is preferably about 200 mm to 1000 mm, the outer diameter thereof is preferably about 1.7 mm to 6.0 mm, and the inner diameter thereof is preferably about 1.0 mm to 5.0 mm.

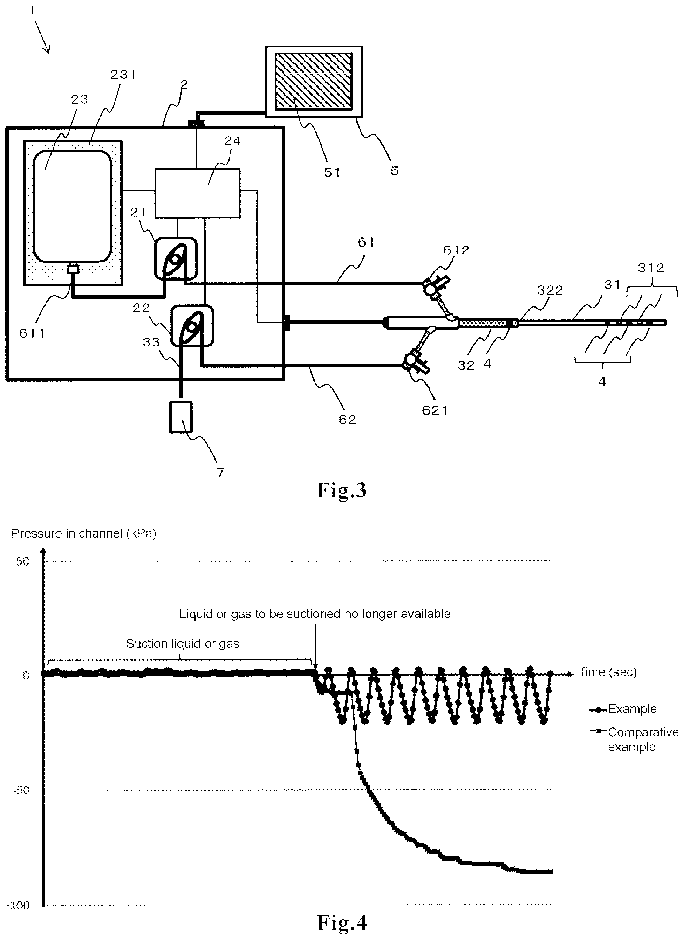

In an embodiment other than the first embodiment, as illustrated in Fig. 3, the first tube 31 may be inserted into the second tube 32, the inner side surface of the second tube 32 and the outer side surface of the first tube 31 may be partially coupled via a coupling section, which is not illustrated, and the first tube 31 and the second tube 32 may thereby be integrated with each other.

In an embodiment other than the first embodiment, the first tube 31 may be inserted into the second tube 32, and a shaft may be inserted into the first tube 31. In this case, a space between the inner surface of the first tube 31 and the outer surface of the shaft serves as the first channel 311, and a space between the inner surface of the second tube 32 and the outer surface of the first tube 31 serves as the first channel 321. With the above configuration, such a structure can be employed that the proximal end side of the second tube 32 is relatively rigid and the second tube 32 gradually becomes flexible toward the shaft on the distal end side. In this way, insertability of the shaft, the first tube 31, and the second tube 32 into the esophagus is improved.

The third tube 33 is a tube for delivering the liquid from the tube 62 to the waste liquid section 7. The waste liquid section 7 is a site for storing the unnecessary liquid after the liquid is suctioned from the body.

The third tube 33 is connected to the tube 62, and the second channel 321 formed in the tube 62 is connected to the outside via the third channel 331. Any flexible material may be used for the third tube 33. For example, a thermoplastic resin such as polyvinyl chloride, polyurethane, or silicone can be used.

The temperature sensor 4 is a member used to measure the internal temperature of the biological lumen, is arranged in the first tube 31, and is preferably arranged on the outer surface of the first tube 31. The one or more temperature sensors 4 are attached to the distal end side of the first tube 31. In order to allow measurement of the internal temperature in a larger area inside the biological organ, a plurality of the temperature sensors 4 are preferably provided. Any material having excellent thermal conductivity may be used for the temperature sensor 4, and such a material preferably has a radiographic contrast property in order to measure a temperature of a position in the vicinity of the ablation site.

In an embodiment other than the first embodiment, in the case where the shaft is inserted into the inside of the first tube 31, the temperature sensor 4 is preferably arranged on an outer surface of the shaft.

The temperature sensor 4 is arranged to the second tube 32 and is preferably arranged on the outer surface of the second tube 32. With the above configuration, in the case where temperature fluctuations are detected before and after the injection of the liquid in the liquid storage section 23 into the biological lumen, it is possible to determine to which part in the biological lumen the liquid has reached. Thus, it is also possible to determine a suction start before aspiration occurs due to inflow of the injected liquid into the trachea, for example.

The monitor 5 can display internal temperature information of the living body detected by the temperature sensor 4, as a digital number, bar graph, or trend graph. The monitor 5 has a function by which, when the temperature in the biological organ exceeds a preset threshold, a display color is changed and a temperature change can be presented as visual information to an operator. For example, the monitor 5 may be set such that the color is changed from a cold color to a warm color as the temperature is increased from a low temperature to a high temperature. In this way, the temperature detected by each of the temperature sensors 4, which are arranged in a longitudinal direction in the living body, can be checked as the temperature at a corresponding position in the living body. Thus, the operator can perceive which site of the biological organ has a higher temperature than the others. In addition, since the temperature is presented not only by the display of the digital number but also by the bar graph, the temperature can be compared among different sites in the living body. Furthermore, it is possible to visually inform the operator of the fact that the internal temperature of the living body has been increased with an increase in the risk of damaging the biological organ.

The monitor 5 preferably has a function to present, to the operator, information on the operation for delivering and suctioning the liquid, information on an error that has occurred to the system and an alert including an alarm, and operation information such as an operation time and amounts of the delivered liquid and the suctioned liquid. In this way, it is possible to visually or aurally inform the operator of an operation status, a malfunctioning condition, and a dangerous condition.

The monitor 5 preferably includes a touch-screen display 51 with which various parameters related to the system actuation can be input, and with which the input parameters can be transmitted to the control section 24 of the *in-vivo* temperature control device 2. This enables the operator at a distant location to start or stop the operation of the *in-vivo* temperature control device 2 and to set and change the various parameters.

The tube 61 is a tube for delivering the liquid from the liquid storage section 23 to the first tube 31 via the liquid injection pump 21, and the first channel 311, through which the liquid flows, is formed in the injection tube 61.

The tube 61 includes a liquid supply port 611 for connection to the liquid storage section 23 and a connection port 612 for connection to the first tube 31.

The liquid supply port 611 may be in any form as long as the liquid can be supplied from the liquid storage section 23 into the injection tube 61. In the first embodiment, since the liquid storage section 23 is the infusion bag, the liquid supply port 611 is preferably a needle capable of piercing the infusion bag.

The connection port 612 may be in any form as long as the connection port 612 can be connected to the injection tube 61. However, the connection port 612 is preferably a three-way stopcock. In this case, when the *in-vivo* temperature control system malfunctions, the liquid can be injected manually by connecting a syringe or the like.

The tube 62 is a tube for delivering the liquid from the second tube 32 to the third tube 33 via the liquid suction pump 22, and the second channel 321, through which the liquid flows, is formed in the tube 62.

The tube 62 includes a connection port 621 for connection to the second tube 32.

The connection port 621 may be in any form as long as the connection port 621 can be connected to the tube 62. However, the connection port 621 is preferably a three-way stopcock. In this case, when the *in-vivo* temperature control system malfunctions, the liquid can be suctioned manually by connecting a syringe or the like.

The following description is made on specific operation for a time chart illustrating first control operation of the *in-vivo* temperature control system for appropriately controlling the internal esophageal temperature during the treatment of arrhythmia by the catheter ablation procedure.

A plurality of the esophagus temperature thresholds and liquid delivery rates corresponding thereto can be set in advance in the control section 24. Thus, the continuously cooled liquid (cooled water) can be injected into the living body according to the set temperature. In addition, one or more of the following can be set in advance in the control section 24: time to start a cycle of the injection to the suction of the cooled water; a threshold of the esophagus temperature at the start of the suction; an accumulated injection amount with which the suction is started; a liquid suction rate and a liquid suction time. Accordingly, the continuously released liquid can be collected from the living body.

### Step 1: Processing for Comparing Temperature Information with Threshold

After the catheter ablation procedure for ablating cardiac tissue is started in the vicinity of the left atrium, the internal esophageal temperature is gradually increased in the vicinity thereof, and the internal esophageal temperature measured by the temperature sensor 4 is also gradually increased. In the control section 24, the threshold of the internal esophageal temperature at which the delivery of the cooled liquid (cooled water) is started can be set in advance, and processing of comparing the temperature information of the temperature sensor 4 with the threshold is constantly carried out.

### Step 2: Operation for Delivering Liquid (Cooled Water)

When the temperature information detected by at least one of the temperature sensors 4 reaches a first esophagus temperature threshold (first threshold), the control section 24 outputs a drive command at a first delivery rate to the liquid injection pump 21, and the cooled liquid (cooled water) is delivered from the liquid storage section 23 to the first tube 31 via the tube 61. Furthermore, when the internal esophageal temperature is increased as the catheter ablation procedure progresses, and the temperature information of at least one of the measured internal esophageal temperatures reaches a second esophagus temperature threshold (second threshold), the control section 24 outputs the drive command at a second delivery rate to the liquid injection pump 21. Thereafter, the operation proceeds to third setting in response to the increase in the esophagus temperature.

When the internal esophageal temperature becomes lower than an Nth temperature threshold due to the injection of the liquid (cooled water) into the esophagus, control is executed to output the drive command at an N-1th delivery rate to the liquid injection pump 21. Then, when the internal esophageal temperature becomes lower than the first threshold, the delivery of the liquid (cooled water) is stopped. During the ablation procedure, the liquid (cooled water) can be delivered at an appropriate flow rate according to the increase or reduction in the internal esophageal temperature by repeatedly executing the control in step 2. Thus, the inside of the esophagus can be cooled further effectively.

At this time, the delivery rate of the liquid (cooled water) is preferably increased with the increase in the esophagus temperature in a manner that the first delivery rate < the second delivery rate < ... < the Nth delivery rate. More specifically, the delivery rate is preferably set within a range of 1 to 300 mL/min. More preferably, the delivery rate is set to be high (more specifically, 200 mL/min to 300 mL/min) when the internal esophageal temperature exceeds a threshold (for example, as the internal temperature, 40°C is set as the threshold), and the delivery rate is set to be low (more specifically, 1 mL/min to 50 mL/min) until the internal esophageal temperature exceeds the threshold. This reduces the risk of aspiration due to overdose of the liquid (cooled water).

### Step 3: Operation for Suctioning Liquid Delivered into Esophagus

In order to prevent the aspiration caused by the liquid injected into the esophagus, the suction operation is preferably started on a drive record of the liquid injection pump 21. More specifically, it is preferred to start suction of the liquid when an integrated value of the injection amount exceeds a set amount, and the accumulated injection amount with which the suction is started can be set in advance in the control section 24. When the liquid is suctioned from the esophagus, some of the injected liquid may flow into the stomach located downstream, and consequently, the liquid that can be suctioned from the esophagus may become no longer available in the middle of suction. However, since the pressure in the liquid elimination channel is released in conjunction with the drive of the liquid suction pump 22, the suction failure can be prevented.

The operation in step 3 may be linked with the operation in step 2, and the inside of the esophagus can be maintained at an appropriate temperature by repeating steps 1 to 3. Since the first control method is a control method for a case where the internal esophageal temperature is increased due to heat ablation of cardiac muscle by a radiofrequency ablation procedure or the like, the circuit is prepared such that the control section 24 determines that the esophagus temperature has reached the threshold when the esophagus temperature exceeds the threshold. However, in a case of the operative procedure in which the internal esophageal temperature is reduced by a cryoablation procedure or the like, the circuit is prepared such that, in the above-described first control method, the liquid to be injected is replaced with heated water and that the control section 24 determines that the esophagus temperature has reached the threshold when the esophagus temperature becomes lower than the threshold. Then, the heated liquid is eliminated. In this way, the internal esophageal temperature can be controlled.

### EXAMPLE

### (Confirmation of Effect of Operation for Releasing Internal Tube Pressure)

As an example, the *in-vivo* temperature control system 1 according to the first embodiment was manufactured. As a comparative example, an *in-vivo* temperature control system was manufactured that had the three rollers 222 for the liquid suction pump 22 and did not have the unpressed state of the tube 62, that is, the second channel 321 is not provided. Then, confirmation of an effect of releasing the internal tube pressure during suction of the liquid was performed. More specifically, the *in-vivo* temperature control system in the comparative example includes: a catheter having a hole through which the liquid can be delivered or suctioned; an *in-vivo* temperature control device having a pressure sensor and a roller pump with the three rollers for delivering/suctioning the liquid to/from the catheter; a temperature probe having a temperature sensor; and a liquid delivery/suction dual-purpose tube connected to the pressure sensor and connecting the *in-vivo* temperature control device to the catheter.

In order to confirm the effect of the release operation of the internal tube pressure, in the *in-vivo* temperature control system of the example, the liquid and gas (air) were mixed in a closed, simulated esophagus tube with flexibility, and the second tube 32 was then inserted into the tube. At the time, a pressure gauge was connected to measure the pressure in the second channel 322 inside the tube 62. Thereafter, the liquid suction pump 22 was driven to start suctioning the liquid and the gas, and the pressure fluctuations in the second channel 321 were recorded. The results thereof are presented in Fig. 4.

Similarly, in the *in-vivo* temperature control system of the comparative example, the liquid and the gas (air) were mixed in the simulated esophageal tube, and then the liquid delivery/suction dual-purpose tube was inserted thereinto. At the time, the pressure gauge was connected to measure the pressure in the liquid delivery/suction dual-purpose tube. Thereafter, the roller pump was driven to start suctioning the liquid and the gas, and the pressure fluctuations in the liquid delivery/suction dual-purpose tube were recorded. The results thereof are presented in Fig. 4.

It is understood from the results that, in the case of the *in-vivo* temperature control system 1 of the example, when the liquid or the gas to be suctioned is no longer available, the negative pressure is generated in the second channel 321, but the negative pressure is periodically released. Meanwhile, it is understood that, in the *in-vivo* temperature control system of the comparative example, when the liquid or the gas to be suctioned is no longer available, the negative pressure in the tube is not released and is maintained.

These results are examined. The *in-vivo* temperature control system in the comparative example cannot release the negative pressure in the suction channel. Thus, the liquid cannot be suctioned from the inside of the esophagus unless the pressure is released to the outside. On the other hand, the *in-vivo* temperature control system 1 in the example can release the pressure generated in the second channel 321 by the release operation each time. Thus, when the liquid is continuously suctioned from the inside of the esophagus, it is possible to prevent a case where the esophageal wall closes the opening to generate the negative pressure, resulting in the suction failure.

### INDUSTRIAL APPLICABILITY

The present invention provides a system in the medical field, the system being able to automatically control the internal temperature of a body cavity in a case of a change in the internal temperature of the body cavity during the procedure.

### DESCRIPTION OF SYMBOLS

1: *in-vivo* temperature control system, 2: *in-vivo* temperature control device, 4: temperature sensor, 5: monitor, 7: waste liquid section, 21: liquid injection pump, 22: liquid suction pump, 23: liquid storage section, 24:control section, 31: first tube, 32: second tube, 33:third tube, 51: touch-screen display, 61: tube, 62: tube, 221: rotor, 222: roller, 231: Peltier device, 311: first channel, 312: opening, 321: second channel, 322: opening, 331: third channel, 611: liquid supply port, 612: connection port, 621: connection port

## Claims

1. An *in-vivo* temperature control system, comprising:
a first tube provided with a first channel;
a second tube provided with a second channel;
a liquid storage section for storing a liquid;
a liquid injection pump for supplying the liquid from the liquid storage section to the first channel and discharging the liquid into a living body via the first channel;
a liquid suction pump for collecting the liquid in the living body via the second channel; and
a third tube provided with a third channel, one end of which is connected to the liquid suction pump, and the other end of which is opened to the outside,
wherein the liquid suction pump switches operation between discharge operation for discharging the liquid in the living body into the third channel and release operation for releasing the pressure in the second channel to the outside via the third channel.

2. The *in-vivo* temperature control system of claim 1, comprising a shaft inserted into the first tube and protruding from the distal end of the first tube.

3. The *in-vivo* temperature control system of claim 1 or 2, comprising:
a temperature sensor arranged in the first tube, the second tube, or the shaft and capable of measuring a temperature in the living body; and
a control section for controlling the liquid injection pump and the liquid suction pump,
wherein the control section controls the liquid injection pump to discharge the liquid from the first tube into the living body on the basis of the temperature measured by the temperature sensor, and controls the liquid suction pump to switch the operation between the discharge operation and the release operation on the basis of a drive record of the liquid injection pump.

4. The *in-vivo* temperature control system of any one of claims 1 to 3,
wherein the liquid suction pump has a roller pump,
in the discharge operation, the second channel is pressed by rotational drive of a roller to deliver the liquid in the second tube, and
in the release operation, the pressure in the second channel is released to the outside by providing an unpressed state of the second channel during one rotation of the roller.

5. The *in-vivo* temperature control system of any one of claims 1 to 3,
wherein the liquid suction pump has a finger pump including a plurality of fingers,
in the discharge operation, the plurality of fingers sequentially press a fixed range of the second channel to deliver the liquid in the second tube, and
in the release operation, the pressure in the second channel is released to the outside by providing an unpressed state of the second channel during delivery of the liquid by the fingers.
